# EUROPEAN PATENT APPLICATION

(11) **EP 1 481 704 A2**
(43) Date of publication of application: **01.12.2004**
(21) Application number: 04425305.2
(22) Date of filing: 30.04.2004
(51) Int. Cl.: A61M 21/00

(54) **Subliminal learning and healing memory optimizer**

(30) Priority: 30.04.2003 IT MI20030872; 30.04.2003 IT MI20030205 U
(71) Applicant: Onstore S.r.l., 20141 Milano (IT)
(72) Inventor: Piraino, Ernesto, 20141 Milano (IT)
(74) Representative: Siniscalco, Fabio

(57) **Abstract**

The invention concerns a subliminal method memory optimizer (100) comprising means to record a voice message and a transducer (10) to convert said recorded voice message into vibrations, which enable subliminal listening of the message by the user.

The memory optimizer is characterized in that said recording means comprise a memory (6) to memorize said digital data corresponding to said message and in that said memory optimizer (100) further comprises a programmable processor (5) connected to said memory and to said transducer (10) which enables the user to program subliminal listening of the message for a preestablished number of times and at fixed hours of the day.

## Description

The present invention refers to memory optimizers which operate using the subliminal method.

Memory devices, which operate on the basis of the subliminal method, utilizing a bone transducer that enables the transmission of data (information of audio-phonetic nature) to the user by means of acoustic vibrations, are well-known. By means of said methods, the user acquires information subconsciously and through the said bone transducer, with suitable signal intensity, or, alternatively, through a specially manufactured device called "pillow" or "under pillow".

At present, the methods available to produce said learning methods are based on the use of common recorders (to be operated exclusively by the user who must manually operate the function keys) or on the use of specially produced recorders which permit, even if to a limited extent, automatic operation.

In particular, with some devices available on the market, it is possible to record with a microphone on to a magnetic support or to make use of lessons prepared on tape or Compact Disk.

In any case, subliminal memory optimizers available on the market have two substantial limitations. The first is connected to the fact that these conventional memory optimizers are not portable and, therefore, can be used for the most part only in the home. The fact that they can only be used in the home is in contradiction with the very nature of the subliminal method which, necessarily, does not require the user's attention.

Another limitation of conventional memory optimizers is connected to the fact that they require direct operation of the function keys by the user or, in any case, their programming possibilities are insufficient for the requirements of the subliminal method.

In fact, it has been observed that subliminal learning is more efficient during sleep than during other phases of the daily life of each individual. Furthermore, the efficiency of learning also seems to depend on the particular sleep phase during which subliminal "listening" takes place. For example, according to some scientific research underway, the notions imprinted with a determinate signal intensity and timbre during the "deep sleep" phase are more easily acquired than during other sleep phases.

As a result of the absence of and/or insufficient programmability of conventional memory optimizers, the user cannot set the sleep phase during which he wants to listen subliminally and, therefore, these devices are mainly used only during the first few hours of the night and day or other parts of the day which are not entirely suitable for their purposes.

Object of the present invention is to propose a subliminal memory optimizer which is more flexible to use than the above-mentioned conventional memory optimizers, especially as far as portability and programmability is concerned.

The object of the present invention is reached by a memory optimizer as defined in the attached claim 1. Preferred embodiments of the memory optimizer are defined in the dependent claims. Claim 11 is intended for use of the memory optimizer for subliminal listening by the user of messages to be memorized.
A first advantage of this invention is connected to the use of purely digital recording and reproduction techniques (for example, Flash memories) which, avoiding the use of magnetic tapes or Compact Disks, make it possible to obtain a reduction in the overall size, weight and consumption of the memory optimizer, so producing the desired portability.

Portability makes it possible to fully exploit the characteristics of the subliminal method, enabling use of the memory optimizer subject of this invention even during activities which are not only intellectual or during sleep, such as sporting activities, during train journeys or flights, while watching television, etc.

Another advantage is connected to the flexibility of programming (obtained thanks to the use of a microprocessor) which makes it possible to "listen" subliminally to one or more pre-recorded messages (or sequences of messages), a fixed number of times and at fixed hours of the day. Programmed repetition of texts/lessons included in the messages helps to accelerate learning (for example, a foreign language, a university lesson, a speech or an actor's script), exploiting to the fullest the potential of the subliminal method.

Furthermore, programmability can be advantageously used to adjust the timbre and level of the signal and the order in which the sequence of the points to be memorized is reproduced.
Finally, the memory optimizer of this invention can preferably be connected to external equipment on a standard USB (Universal Serial Bus) interface, such as a personal computer, so as to provide new possibilities of use also based on the Internet as a distribution network of information and services.

For a better understanding of the characteristics and advantages of the present invention, some nonlimiting embodiments thereof are described below, with reference to the appended drawing, where:

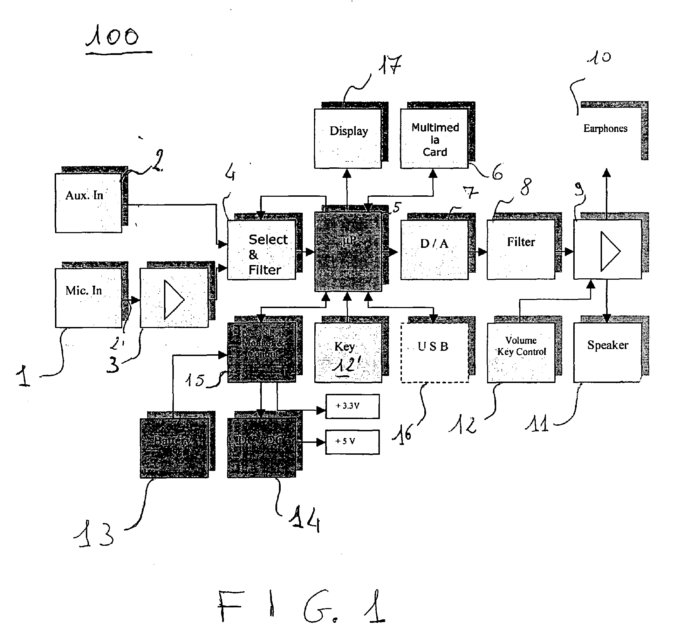

- figure 1 is a block diagram of a subliminal method memory optimizer, according to a preferred embodiment of the invention.

### Audio signal input source.

Figure 1 shows, through a functional block diagram, an embodiment of a subliminal method memory optimizer 100 produced according to this invention.
The subliminal method memory optimizer 100 is provided with an input microphone 1 (Mic. In) and an auxiliary input 2 (Aux. In). A message to be recorded, emitted from an input audio source (not illustrated) can be acquired by selecting either the input microphone 1 or the auxiliary input 2.

The auxiliary input 2 provides the possibility of using, as an external source, an external device such as a Compact Disk or audiocassette reader.
The input microphone 1 is connected, by means of a terminal 2', to a suitably adapted input circuit 3 through a signal pre-emphasis and compression stage.

Selection between the input microphone 1 and the auxiliary input 2 can be made by means of an input selector included, according to figure 1, in a functional block 4 (Select & Filter), also comprising a low pass filter connected to the selector block output. The low pass filter has, for example, a cut-off frequency calibrated at 4.6 KHz to eliminate all high frequency components presumably coupled to the feeding circuit of the amplification stage 3.

The output of said filter included in block 4 is then applied to an Analog-Digital converter inside processor 5 (a microcontroller or a microprocessor µP) having, for example, a resolution of 10 bits and a fixed sampling time of approximately 15.5 KHz, easily within double the desired pass band, in order to avoid possible residual disturbance during reconversion.

### Audio data management.

The digital data resulting from the analog-digital conversion performed by the converter present in the microcontroller 5, are advantageously compressed by means of a CELP and Huffman coding, which makes it possible to optimize the quality/compression factor ratio for voice signal compression applications. The microcontroller 5 is connected to a flash memory 6 or a memory-card (Multimedia card) which memorizes the digitally converted data which are associated to a descriptor.

### Audio signal output

The subliminal method memory optimizer 100 comprises a digital-analog converter 7 (D/A) (for example, external to the microcontroller 5) whose output stage is connected to the input of a further low pass filter 8 (Filter) having, for example, a cut-off frequency equal to 4.2 KHz.

The output of said further low pass filter 8 is connected, by means of an output amplifier stage 9, to three different outputs:
a first output which can be connected to a transducer or earphones 10 of the conventional type;
a second output (using, for example, the same output 10) which can be connected to a pillow (not illustrated) of the type used for subliminal method memory optimizers, such as those commercialized by Onstore S.r.l. (Milan);
a third output which can be connected to an internal speaker 11 (Speaker).

Advantageously, two keys for volume adjustment are provided which act on a volume adjustment circuit block 12 (Volume Key Control) connected to the output amplification stage 9 in order to regulate the volume of the internal speaker 11. The output amplification stage 9 can also be disabled during operations other than reproduction, and during reproduction when connectors are plugged in on the other outputs in order to reduce consumption.

### Power Supply Control:

The subliminal method memory optimizer 100 is provided with batteries 13 (Battery block), for example, two 1.5V AAA stylus batteries. For internal stages which require more than 3 Volts, a DC/DC converter 14 is used which provides a voltage of +5 Vcc.

The subliminal method memory optimizer further comprises a monitoring circuit 15 (Power Supply Monitor & Control) for the battery level 13, which is connected to the processor 15 and indicates the battery charge.

### USB Interface

Furthermore, the subliminal method memory optimizer 100 is provided with a USB type interface 16 for connection to external devices.

### Management Interface

The subliminal method memory optimizer 100 is provided with an adequate housing for the blocks illustrated in figure 1 and is provided with keys (for example, four keys connected to the circuit block 12 "Key", in its turn connected to the processor 5) which make it possible for the user to program with the help of the information appearing on a display 17. A menu appears on the display 17 giving various programming options which can be selected by the user.

During operation, a voice signal is translated by the input microphone 1 which converts it into an electric signal, which, after undergoing amplification in the pre-emphasis block 3, is suitably filtered by the low pass filter in block 4. Alternatively, an analog electric signal corresponding to the message to be recorded and deriving from analog or digital audio supports (such as CD reader, music cassette or mp3) is fed to the auxiliary input 2.

The analog signal is then converted into digital form by the processor 5 and the corresponding digital data are memorized in the flash memory 6. Thanks to special software with which the processor 5 is provided, the user can program (by means of the keys and with the help of the display 17) complete reproduction of the recorded messages.

In particular, it is possible to repeat either a brief message or an entire text/lesson for a number of times to be determined each time programming is carried out and then start up the system at a given hour, so that the user can choose the number of repetitions he requires, on the basis of his learning capacity.

With the use of a flash memory it is possible to listen to the recorded messages whenever desired and to select the segment of interest for reproduction as previously established during programming.

During reproduction, the processor 5 recovers the digital data from the flash memory 6 and sends them to the digital/analog converter 7 and then to the low pass filter 8. After suitable amplification, the analog electric signal is sent to the earphones 10 (or pillow) to emit the vibrations necessary for subliminal listening. The memory optimizer blocks 100 can be provided on a special chip.

A substantial innovation of the present invention is given by the miniaturization of the memory optimizer 100, obtained thanks to the use of the digital memory 6 which has made it possible to avoid the use of bulky magnetic type cassettes and the mechanical device necessary to move the magnetic tape. In this way, the memory optimizer of the invention is portable and the user is not bound to only a static form of use.

The memory optimizer according to the invention can advantageously be used for learning purposes since it accelerates the learning process of a notion by means of repetition of a text or a lesson through the earphones, while the user is busy in other activities.

Furthermore, the memory optimizer according to the invention has advantageous applications in therapeutic/clinical activities in those cases where the patient is in a coma and is waiting to "wake up".

In fact, the memory optimizer according to the invention makes it possible for other people (family members or health personnel) to program the memory optimizer in such a way that the patient can listen to the repeated messages for as long as necessary.

According to another advantageous application, the memory optimizer according to the invention can be used for subliminal listening and, therefore, for subliminal memorizing, of messages difficult for the user to accept. For example, messages which stimulate the user to stop smoking, eat less or drink alcohol in moderation can be recorded on the memory 6 and reproduced through the earphones 10 or the pillow.

## Claims

1. Subliminal memory optimizer (110) comprising:
- means to record a voice message,
- a transducer (10) to convert said recorded voice message into vibrations which enable subliminal listening of the message by the user,
**characterized in that** said recording means comprise a memory (6) to memorize digital data corresponding to said message, and **in that** said memory optimizer (100) further comprises a programmable processor (5) connected to said memory and to said transducer (10) which makes it possible for the user to program subliminal listening of the message for a preestablished number of times and at fixed hours of the day.

2. Memory optimizer according to claim 1, comprising:
- at least one input (1, 2') for an analog signal corresponding to the message to be recorded,
- an analog-digital converter to convert the analog signal in said digital data, said processor (5) enabling memorization of the digital data in the memory (6).

3. Memory optimizer according to claim 1, where said memory is a flash memory.

4. Memory optimizer according to claim 1, where said memory is a memory card.

5. Memory optimizer according to claim 2, further comprising a microphone (1) connected to said at least one input (2') for the analog signal.

6. Memory optimizer according to claim 2, where said at least one input (2) can be connected to an external device which supplies said analog signal.

7. Memory optimizer according to claim 2, further comprising a digital-analog converter (7) connected to a processor (5) to convert the digital data memorized in the memory (6) into a further analog signal and to supply said further signal to said transducer.

8. Memory optimizer according to at least one of the previous claims, where said transducer is a pair of earphones or a transducer pillow.

9. Memory optimizer according to claim 1, configured in such a way as to be portable and comprising batteries for power supply.

10. Memory optimizer according to claim 2, including a USB port for the connection of said processor to an external device, such as, for example, a personal computer.

11. Use of the memory optimizer constructed according to at least one of the previous claims for subliminal listening of messages to be memorized by the user.
